# EUROPEAN PATENT APPLICATION

(11) **EP 4 360 637 A2**
(43) Date of publication of application: **01.05.2024**
(21) Application number: 24161247.2
(22) Date of filing: 11.08.2020
(51) Int. Cl.: A61K 31/635

(54) **AMMONIA AS A PROCESSING AID FOR SPRAYED SOLID DISPERSIONS**

(30) Priority: 15.08.2019 US 201962887471 P
(62) Divisional of application: 20767607.3
(71) Applicant: Capsugel Belgium NV, 2880 Bornem (BE)
(72) Inventor: MILLER, Warren K., Bend, OR 97703 (US); MORGEN, Warren K., Bend, OR 97703 (US)
(74) Representative: De Clercq & Partners

(57) **Abstract**

Ammonia is used as a processing aid in a method for forming a sprayed solid dispersion comprising an active agent, wherein the active agent, in a free acid form, has a pKa ≤ 7 and a solubility ≤ 40 mg/mL in the spray solvent. Ammonia is subsequently removed from the sprayed solid dispersion. Sprayed solid dispersions formed by the disclosed method are also disclosed.

## Description

### CROSS-REFERENCE TO RELATED APPLICATION

This application claims the benefit of the earlier filing date of U.S. Provisional Application No. 62/887,471, filed August 15, 2019, which is incorporated by reference in its entirety herein.

### FIELD

This disclosure concerns a method for using ammonia as a processing aid when forming a sprayed solid dispersion and subsequently removing ammonia from the sprayed solid dispersion. Sprayed solid dispersions formed by the disclosed method are also encompassed by the disclosure.

### SUMMARY

Embodiments of a method for using ammonia as a processing aid when forming a sprayed solid dispersion are disclosed, as well as embodiments of sprayed solid dispersions formed by the method. In some embodiments, a method for preparing a sprayed solid dispersion includes (i) combining an active agent, a dispersion polymer, an amount of ammonia, and a solvent to form a spray solution; (ii) spraying the spray solution to form a sprayed solid dispersion comprising the active agent, the dispersion polymer, and ammonia; and (iii) removing residual ammonia from the sprayed solid dispersion to form a product comprising a solid dispersion of the active agent and the dispersion polymer, the product comprising ≤ 500 ppm ammonia. In any of the foregoing embodiments, at least 90 wt% of the active agent in the product may be in the free acid form.

In some embodiments, the solvent comprises a C₁-C₃ alkanol; the active agent, in a free acid form, has a pKa ≤ 7 and a solubility ≤ 40 mg/mL in the solvent; and the amount of ammonia is sufficient to solubilize the active agent in the solvent. The solvent may comprise the C₁-C₃ alkanol and a co-solvent.

In any of the foregoing embodiments, the amount of ammonia may be at least 0.95 molar equivalents relative to a sum of acid groups on the active agent and any acid groups on the dispersion polymer, such as from 0.95 to 10 molar equivalents relative to a sum of acid groups on the active agent and any acid groups on the dispersion polymer and other excipients, if present. In some embodiments, the amount of ammonia is from 0.95 to 4 molar equivalents relative to a sum of acid groups on the active agent and any acid groups on the dispersion polymer and other excipients, if present. In certain embodiments, the amount of ammonia is at least 0.95 molar equivalents relative to a sum of acid groups on an amount of the active agent that exceeds the free acid form solubility of the active agent in the solvent and any acid groups on the dispersion polymer.

In any of the foregoing embodiments, the active agent may have a solubility in the spray solution at least 2-fold higher than a solubility of a free acid form of the active agent in the solvent without ammonia. In any of the foregoing embodiments, the spray solution may have a dissolved solids content within a range of 2-40 wt%, wherein 0.5-95 wt% of the dissolved solids is the active agent. In some embodiments, the spray solution comprises 0.5-20 wt% of the active agent.

In any of the foregoing embodiments, removing residual ammonia may include heating the sprayed solid dispersion at a temperature within a range of 30-70 °C and a relative humidity (RH) within a range of 10-75% for a sufficient period of time to form the product comprising ≤ 500 ppm ammonia. In some embodiments, (i) the period of time is from 2 to 60 hours, or (ii) the temperature is within a range of 40-60 °C, or (iii) the RH is within a range of 15-50%, or (iv) any combination of (i), (ii), and (iii). In any of the foregoing embodiments, removing residual ammonia may further include blowing a sweep gas across the sprayed solid dispersion while heating the sprayed solid dispersion at the temperature and RH.

Embodiments of a sprayed solid dispersion prepared by the disclosed methods comprise at least 0.5 wt% of the active agent and ≤ 500 ppm ammonia. In some embodiments, the sprayed solid dispersion comprises 0.5-95 wt% of the active agent, wherein at least 90 wt% of the active agent is amorphous, the dispersion polymer, and ≤ 50 ppm ammonia.

The foregoing and other objects, features, and advantages of the invention will become more apparent from the following detailed description, which proceeds with reference to the accompanying figures.

### BRIEF DESCRIPTION OF THE DRAWINGS

FIG. 1 is a powder X-ray dispersion (PXRD) spectrum of a spray-dried dispersion (SDD) of piroxicam and PVP-VA 64 after drying at 50 °C, 30% relative humidity for 48 hours.
FIG. 2 is a PXRD spectrum of a SDD of piroxicam and HPMCAS after drying at 50 °C, 30% relative humidity for 48 hours.

### DETAILED DESCRIPTION

Methods for forming sprayed solid dispersions (e.g., spray-dried dispersions, spray-layered dispersions) comprising an active agent and a dispersion polymer generally comprise spraying a solution comprising the active agent, the dispersion polymer, and a solvent. A problem that frequently arises during the spray-drying of active agents, such as active pharmaceutical ingredients (APIs), is that low solubility of the active agent in preferred volatile organic spray solvents, such as acetone and methanol, can adversely affect manufacturability and product quality. Specifically, low dissolved solids concentration in the spray solution can result in unacceptably low manufacturing throughput and poor particle properties. In some cases, the solubility in the spray solvent is so low that commercial manufacture of spray-dried intermediates, such as spray-dried dispersions (SDDs) or spray-layered dispersions (SLDs), is not economically feasible and/or cannot be performed without using extraordinary means such as high temperature and/or dilute solutions with long spray times. Increasing temperatures, however, can lead to thermal and/or chemical degradation of some active agents. Long sprays of dilute solutions tie up equipment for extended periods of time, lowering throughput and profits, and potentially adversely affecting particles properties. Poorly soluble active agents often have a strong tendency to crystallize and have high melting points, contributing to their low organic solvent solubility. Additionally, many of these active agents exist in different polymorphs and tend to recrystallize or convert to the lowest energy (least soluble) polymorph in the spray solvent. If the low solubility active is a weak acid (pKa 3-7), then one solution to the low organic solubility of the active is to ionize the active with a base to increase the active solubility in a polar volatile organic solvent such as methanol. A process has been described previously (US 8,372,836) in which such a base comprises a non-volatile counter ion that is not removed from the spray dried material, and becomes part of the product as the salt of the acid, for example, the potassium salt. Two key limitations of this approach are that 1) it reduces the potency of the formulation by adding the nonvolatile counterion, and 2) the presence of the nonvolatile counterion can negatively affect the physical stability of the product, e.g. through the increased hygroscopicity of the salt form of the active relative to the free acid form.

Embodiments of the disclosed process mitigate one or more of these problems. As disclosed herein, a volatile base, ammonia, is used to form an ammonium salt with a weak-acid active agent, yielding a several-fold higher concentration of dissolved active in a volatile solvent. This ammonium salt of the active can be prepared in the presence of a dispersion polymer and other excipients, and then sprayed (e.g., spray dried or spray layered) to form a sprayed dispersion (SD). During the spraying process, some of the ammonia is removed, regenerating some of the free acid form of the active agent. During secondary drying of the SD to remove solvent, additional ammonia is removed, but not to the desired extent. In some embodiments, the nearly complete removal of ammonia, and thus nearly complete regeneration of the active in its free acid form, typically utilizes extended processing times and/or more aggressive drying conditions (e.g. relative saturation of a solvent using solvent-assisted drying, relative humidity, and/or elevated temperatures) than would typically be used in the absence of the added base. In some cases, increased temperature and humidity with or without extended processing times will remove nearly all of the ammonia and regenerate nearly all of the active agent as the free acid. The time, temperature, and/or humidity conditions used to remove nearly all of the ammonia may depend at least in part on the pKa of the active agent, the type of dispersion polymer and other excipients used, the mass ratio of these materials, and/or, potentially, the particle morphology.

### I. Definitions and Abbreviations

The following explanations of terms and abbreviations are provided to better describe the present disclosure and to guide those of ordinary skill in the art in the practice of the present disclosure. As used herein, "comprising" means "including" and the singular forms "a" or "an" or "the" include plural references unless the context clearly dictates otherwise. The indefinite article "a" or "an" thus usually means "at least one." The term "or" refers to a single element of stated alternative elements or a combination of two or more elements, unless the context clearly indicates otherwise.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting. Other features of the disclosure are apparent from the following detailed description and the claims.

The disclosure of numerical ranges should be understood as referring to each discrete point within the range, inclusive of endpoints, unless otherwise noted. Unless otherwise indicated, all numbers expressing quantities of components, molecular weights, percentages, temperatures, times, and so forth, as used in the specification or claims are to be understood as being modified by the term "about." The term "about" as used in the disclosure of numerical ranges indicates that deviation from the stated value is acceptable to the extent that the deviation is the result of measurement variability and/or yields a product of the same or similar properties. Accordingly, unless otherwise implicitly or explicitly indicated, or unless the context is properly understood by a person of ordinary skill in the art to have a more definitive construction, the numerical parameters set forth are approximations that may depend on the desired properties sought and/or limits of detection under standard test conditions/methods as known to those of ordinary skill in the art. When directly and explicitly distinguishing embodiments from discussed prior art, the embodiment numbers are not approximates unless the word "about" is recited.

Although there are alternatives for various components, parameters, operating conditions, etc. set forth herein, that does not mean that those alternatives are necessarily equivalent and/or perform equally well. Nor does it mean that the alternatives are listed in a preferred order unless stated otherwise.

Definitions of common terms in chemistry may be found in Richard J. Lewis, Sr. (ed.), Hawley's Condensed Chemical Dictionary, published by John Wiley & Sons, Inc., 2016 (ISBN 978-1-118-13515-0).

**Active agent:** As used herein, the term "active agent" refers to a component that exerts a desired physiological effect on a mammal, including but not limited to humans. Synonymous terms include "active ingredient," "active substance," "active component," "active pharmaceutical ingredient," and "drug."

**Amorphous:** Substantially non-crystalline (e.g., at least 95% non-crystalline). Amorphous solids lack a definite crystalline structure and a sharp, well-defined melting point; instead, an amorphous solid melts gradually over a range of temperatures.

**Dispersion:** A system in which particles are distributed in a continuous phase of a different composition. A **solid dispersion** is a system in which at least one solid component is distributed in another solid component.

**Excipient:** A physiologically inert substance that is used as an additive in a pharmaceutical composition. As used herein, an excipient may be incorporated within particles of a pharmaceutical composition, or it may be physically mixed with particles of a pharmaceutical composition. An excipient can be used, for example, to dilute an active agent and/or to modify properties of a pharmaceutical composition. Examples of excipients include but are not limited to binders, fillers, diluents, disintegrants, glidants, surfactants, coatings, coloring agents, flavorants, and combinations thereof. The term "excipient" does not include the dispersion polymers disclosed herein.

**Glass transition temperature,** *T_{g}*: The temperature at which an amorphous solid, such as glass or a polymer, becomes brittle or strong on cooling, or soft or pliable on heating. *T_{g}* can be determined, for example, by differential scanning calorimetry (DSC). DSC measures the difference in the amount of heat required to raise the temperature of a sample and a reference as a function of temperature. During a phase transition, such as a change from an amorphous state to a crystalline state, the amount of heat required changes. For a solid that has no crystalline components, a single glass transition temperature indicates that the solid is homogeneous or a molecular dispersion. In general, when a glass is tested by increasing the temperature of the sample at a constant rate, typically 1 to 10 °C/min, a relatively sharp increase in heat capacity will be observed in the vicinity of the T_{g}. T_{g} can also be measured by a dynamic mechanical analyzer (DMA), a dilatometer, or by dielectric spectroscopy. T_{g} values measured by each technique may vary, but generally fall within 10-30 °C of one another. For example, the T_{g} measured by DMA is often 10-30 °C higher than the T_{g} measured by DSC. **Dry glass transition temperature** refers to the *T_{g}* measured when the solid is equilibrated at a relative humidity ≤ 5%.

**Relative humidity (RH):** A measure of the amount of water in air compared with the amount of water the air can hold at a particular temperature.

**SD:** Sprayed solid dispersion.

**SDD:** Spray-dried dispersion.

**SLD:** Spray-layered dispersion.

**Solubilize:** To make soluble or increase the solubility of.

**Solution:** A homogeneous mixture composed of two or more substances. A solute (minor component) is dissolved in a solvent (major component). In contrast to a suspension, light passes through a solution without scattering from solute particles.

**Spray solution:** As used herein, the term "spray solution" refers to a fluid formed by dissolving an active agent and a dispersion polymer in a solvent and an amount of ammonia. In the case of the active agent, the term "dissolved" has the conventional meaning, indicating that the active agent has gone into solution when combined with the solvent and the amount of ammonia. In the case of dispersion polymers, the term "dissolved" can take a broader definition. For some dispersion polymers, the term dissolved can mean that the dispersion polymer has gone into solution and has dissolved in the conventional sense, or it can mean that the dispersion polymer is dispersed or highly swollen with the solvent such that it acts as if it were in solution, or it can mean that a portion of the dispersion polymer molecules are in solution and the remaining dispersion polymer molecules are dispersed or highly swollen with solvent. Any suitable technique may be used to determine if the active agent and dispersion polymer are dissolved. Examples include dynamic or static light scattering analysis, turbidity analysis, and visual observations.

**Volatile organic solvent:** An organic solvent with a boiling point of 150 °C or less. Volatile organic solvent include, but are not limited to, methanol, ethanol, propanol isomers, butanol isomers, 1-pentanol, 2-methyl-1-propanol, acetic acid, formic acid, certain ketones (e.g., acetone, methyl ethyl ketone), certain esters (e.g., methyl acetate, ethyl formate, ethyl acetate), C₄-C₇ alkanes, and the like.

### II. Spray Processes

Ideally, a spray solution has a dissolved solids content (e.g., active agent and dispersion polymer) of 10-15 wt% at ambient temperature. It is often advantageous to prepare sprayed solid dispersions (SDs) comprising an active agent and a dispersion polymer, with the SD having a high loading of the active agent, such as an active agent content of at least 25 wt%.

In some embodiments, the active agent is a weakly acidic active agent (such as an active agent with a pKa in the range of 3-7 in its free acid form). Many weakly acidic active agents are poorly soluble in common organic spray solvents, including volatile organic solvents such as C₁-C₅ alkanols e.g., methanol, ethanol, n-propanol, isopropanol, etc. As used herein, "poorly soluble" means the active agent in a free acid form has a solubility ≤ 40 mg/mL in one or more C₁-C₅ alkanols, such as a solubility ≤ 30 mg/mL, ≤ 20 mg/mL, or ≤ 10 mg/mL in one or more C₁-C₅ alkanols. In some embodiments, the active agent in a free acid form has a solubility ≤ 40 mg/mL in one or more C₁-C₃ alkanols, such as a solubility ≤ 30 mg/mL, ≤ 20 mg/mL, or ≤ 10 mg/mL in one or more C₁-C₃ alkanols. For example, the active agent may have a solubility ≤ 40 mg/mL in methanol, ethanol, n-propanol, and/or isopropanol at ambient or room temperature, where ambient temperature is within a range of 20-25 °C. In certain examples, the active agent has a solubility ≤ 10 mg/mL in methanol, ethanol, n-propanol, and/or isopropanol at ambient temperature

The solubility of the active agent may be increased by forming an ammonium salt of the active agent in the spray solution. In some embodiments, a weakly acidic active agent is solubilized in a C₁-C₅ alkanol, by converting the active agent to an ammonium salt of the active agent. In certain embodiments, the C₁-C₅ alkanol is a C₁-C₃ alkanol or a combination of C₁-C₃ alkanols. Exemplary alkanols include methanol, ethanol, n-propanol, isopropanol, or any combination thereof. However, the presence of ammonia in the final product frequently is undesirable. The ammonia may impart an unpleasant odor to the product. In some instances, ammonia present in the product may, over time, degrade the active agent and/or dispersion polymer.

A sprayed solid dispersion may be prepared by (i) combining an active agent, a dispersion polymer, an amount of ammonia, and a solvent to form a spray solution, (ii) spray drying the spray solution to form a SD comprising the active agent, the dispersion polymer, and ammonia, and (iii) removing residual ammonia from the SD to form a product comprising a solid dispersion of the active agent and the dispersion polymer, the product comprising ≤ 500 ppm ammonia, such as ≤ 300 ppm, ≤ 200 ppm, ≤ 100 ppm, or even ≤ 50 ppm ammonia. Unless otherwise specified, "ppm" as used herein refers to parts per million by weight. In certain embodiments, the product comprises from 1-500 ppm, such as 1-300 ppm, 1-200 ppm, 1-100 ppm, 1-50 ppm, or 1-25 ppm ammonia. The ammonia content may be determined by any suitable method, such as by use of an ammonia ion selective electrode. In some embodiments, the solid dispersion is a solid amorphous dispersion.

In some embodiments, the solvent comprises a C₁-C₃ alkanol or a combination of C₁-C₃ alkanols, such as the solvent comprises methanol, ethanol, n-propanol, isopropanol, or any combination thereof. In certain embodiments, the solvent further comprises a co-solvent. Exemplary co-solvents include, but are not limited to, water, tetrahydrofuran, ethyl acetate, methyl acetate, 1,3-dioxolane, acetone, methyl ethyl ketone, or any combination thereof. The co-solvent may facilitate dissolution of the active agent, the dispersion polymer, or both. However, the amount of the C₁-C₃ alkanol in the mixed solvent is sufficient to allow use of ammonia to form a salt of the active agent. In some embodiments, the solvent comprises ≤ 50 wt% of one or more co-solvents, such as ≤ 40 wt%, ≤ 30 wt%, ≤ 20 wt%, or ≤ 15 wt% of one or more co-solvents with the remainder being one or more C₁-C₃ alkanols. For instance, the solvent may comprise from 0.01-50 wt% co-solvent, such as from 0.1-40 wt%, 1-30 wt%, 2-20 wt%, or 5-15 wt% of one or more co-solvents. In some embodiments, the co-solvent is water, such as ≤ 30 wt% water, ≤ 20 wt% water, or ≤ 15 wt% water, with the remainder being one or more C₁-C₃ alkanols. In certain embodiments, the solvent comprises 0.01-30 wt% water, 0.1-30 wt% water, 1-30 wt% water, 10-30 wt% water, or 10-20 wt% water. In some examples, water is included when the dispersion polymer comprises hydroxypropyl methyl cellulose (HPMC). In some embodiments, the co-solvent is tetrahydrofuran (THF), such as ≤ 30 wt% THF, ≤ 20 wt% THF, or ≤ 15 wt% THF, with the remainder being one or more C₁-C₃ alkanols. In some examples, THF is included when the dispersion polymer comprises cellulose acetate phthalate (CAP). In certain embodiments, the solvent comprises 0.01-30 wt% THF, 0.1-30 wt% THF, 1-30 wt% THF, 10-30 wt% THF, or 20-30 wt% THF. For instance, the solvent may comprise methanol and THF in a weight ratio of 8:2 or 7:3 when the dispersion polymer comprises CAP.

The active agent, in a crystalline or free acid form, has a pKa ≤ 7 and a solubility ≤ 40 mg/mL in the solvent, such as ≤ 30 mg/mL, ≤ 20 mg/mL, or ≤ 10 mg/mL. In some embodiments, the free acid form of the active agent has a pKa within a range of 3-7. The active agent may have a free acid form solubility within a range of 0.0001 mg/mL to 40 mg/mL in the solvent, such as a free acid form solubility with a range of 0.001-40 mg/mL, 0.001-30 mg/mL, 0.001-20 mg/mL, 0.001-10 mg/mL, 0.01-10 mg/mL, 0.01-5 mg/mL, 0.01-2.5 mg/mL, or even 0.01-1 mg/mL in the solvent.

The amount of ammonia is sufficient to solubilize the active agent in the solvent, i.e., to form a solution of the active agent in the solvent. However, excess ammonia may degrade certain active agents and/or dispersion polymers. Thus, the amount of ammonia is not so great as to degrade the active agent or the dispersion polymer. In some embodiments, the active agent and the solvent are combined to form a suspension or slurry, and sufficient ammonia is added to dissolve the active agent. In an independent embodiment, ammonia and the solvent are combined, and the active agent is subsequently added to form a solution. In some embodiments, the amount of ammonia is at least 0.95 molar equivalents relative to a sum of acid groups on the active agent. However, the amount of ammonia may be significantly greater, and is essentially unlimited, if the additional ammonia does not degrade the active agent or the dispersion polymer. For instance, in some examples, the amount of ammonia may be up to 10 molar equivalents relative to the sum of acid groups on the active agent. In certain embodiments, the amount of ammonia in the spray solution is 0.95-10 molar equivalents relative to a sum of acid groups on the active agent, such as 0.95-5, 0.95-4, 0.95-3, 0.95-2, 0.95-1.5, or 0.95-1.1 molar equivalents relative to the sum of acid groups on the active agent.

The mass of added ammonia used to form the salt of the active agent is correlated to the molecular weight of the active agent. For example, when forming a sprayed dispersion including 100 g of an active agent with a molecular weight of 250 g/mol, one equivalent of ammonia would be 6.8 g. However, when forming a sprayed dispersion including 100 g of an active agent with a molecular weight of 500 g/mol, one equivalent of ammonia would be only 3.4 g.

Advantageously, in some embodiments, addition of ammonia increases the solubility of the active agent in the solvent at least 2-fold. In certain embodiments, ammonia increases the active agent solubility at least 3-fold, at least 4-fold, at least 5-fold, at least 10-fold, at least 20-fold, at least 30-fold, or even at least 40-fold compared to the crystalline (free acid form) solubility of the active agent in the solvent without ammonia.

In some embodiments, however, it may be beneficial or desirable to limit the amount of excess ammonia. Excess ammonia may be limited, for example, when excess ammonia degrades the active agent and/or the dispersion polymer. In such instances, an amount of *undissolved* active agent may be determined or calculated, and the amount of ammonia added may be 0.95-1.1 molar equivalents relative to a sum of acid groups on an amount of the active agent that exceeds the free acid form solubility of the active agent in the solvent, i.e., the *undissolved* amount of the active agent. For example, an active agent X may have a free acid form solubility of 5.0 mg/mL in methanol and a concentration of 20.0 mg/mL may be desired for spray drying. Since 25% of the active agent X has dissolved in the methanol, the amount of ammonia to dissolve the remainder of the active agent X may be 75% of that calculated based on the total concentration (20 mg/mL) of the active agent X. Thus, the amount of ammonia may be ~0.75 molar equivalents relative to sum of acid groups on 20 mg/mL of the active agent X, or 0.95-1.1 molar equivalents relative to a sum of acid groups on 15 mg/mL of the active agent X.

A dispersion polymer is added to the solution to form the spray solution. In some embodiments, the dispersion polymer is soluble in the solvent and has gone into solution. In certain embodiments, the dispersion polymer is dispersed or highly swollen with the solvent such that it acts as if it were in solution. In some instances, a portion of the dispersion polymer molecules are in solution and the remaining dispersion polymer molecules are dispersed or highly swollen with the solvent. The dispersion polymer may have a dry glass transition temperature T_{g} > 60 °C. The dispersion polymer may be added prior to or after addition of ammonia. In certain instances, two or more dispersion polymers are added to the solution to form the spray solution. Some dispersion polymers include one or more acid groups. In such instances, the amount of ammonia added may be at least 0.95 molar equivalents relative to a sum of acid groups on the active agent plus any acid groups on the dispersion polymer, or dispersion polymers, to ensure that sufficient ammonia is present to fully solubilize the active agent. Excess ammonia may be utilized so long as the amount of ammonia is not sufficient to degrade the active agent and/or the dispersion polymer. In some embodiments, the amount of ammonia is 0.95-10 molar equivalents relative to a sum of acid groups on the active agent plus any acid groups on the dispersion polymer, such as 0.95-5, 0.95-4, 0.95-3, 0.95-2, 0.95-1.5, or 0.95-1.1 molar equivalents relative to the sum of acid groups on the active agent plus any acid groups on the dispersion polymer. Alternatively, where it is beneficial or desired to limit the amount of excess ammonia, the amount of ammonia added may be 0.95-1.1 molar equivalents relative to a sum of acid groups on the *undissolved* amount of the active agent plus any acid groups on the dispersion polymer.

The determined amount of ammonia is added to the solvent or to a slurry or suspension comprising the solvent and the active agent. The dispersion polymer may be added before or after addition of ammonia. In some embodiments, the ammonia is added an alcoholic ammonia solution, such as ammonia in methanol, ethanol, or propanol. In an independent embodiment, an aqueous solution of ammonia in water (i.e., ammonium hydroxide) is added. In another independent embodiment, ammonia gas is added.

In some embodiments, the spray solution further comprises one or more excipients. As used herein, the term excipient does not include the dispersion polymer(s). Advantageously, any excipients are soluble in the spray solution. Excipients may be added prior to or after addition of ammonia. In some embodiments, up to 25 wt%, up to 50 wt%, up to 75 wt%, up to 90 wt%, or even up to 95 wt% of the dissolved solids are provided by one or more excipients. For example, the dissolved solids may include 0.01-95 wt%, 0.5-95 wt%, 1-95 wt%, 10-95 wt%, 25-95 wt%, 50-95wt%, 75-95 wt%, 0.5-90 wt%, 0.5-75 wt%, 0.5-50 wt%, or 0.5-25 wt% excipients. In some embodiments, the amount of ammonia is 0.95-4 molar equivalents relative to a sum of acid groups on the active agent plus any acid groups on the dispersion polymer and any acid groups on the one or more excipients, such as 0.95-3, 0.95-2, 0.95-1.5, or 0.95-1.1 molar equivalents relative to the sum of acid groups on the active agent plus any acid groups on the dispersion polymer and any acid groups on the excipient(s). Alternatively, where it is beneficial or desired to limit the amount of excess ammonia, the amount of ammonia added may be 0.95-1.1 molar equivalents relative to a sum of acid groups on the *undissolved* amount of the active agent plus any acid groups on the dispersion polymer and the one or more excipients.

In some embodiments, the spray solution has a dissolved solids content of 2-40 wt% at ambient temperature (e.g., a temperature of 20-25 °C), such as a solids content of 2-30 wt%, 2-25 wt%, 2-20 wt%, 2-15 wt%, 5-15 wt% or 10-15 wt% at ambient temperature, wherein 0.5-95 wt% of the dissolved solids is the active agent. Thus, the spray solution may have a combined active agent and dispersion polymer content of 2-40 wt%, 2-30 wt%, 2-25 wt%, 2-20 wt%, 5-20 wt%, 5-15 wt%, or 10-15 wt%. Alternatively, the spray solution may have a combined active agent, dispersion polymer, and excipient content of 2-40 wt%, 2-30 wt%, 2-25 wt%, 2-20 wt%, 2-15 wt%, 5-15 wt%, or 10-15 wt%. It is often desirable to have an active agent content in the sprayed solid dispersion of at least 25 wt%, such as an active agent content from 25-95 wt% with the balance comprising the dispersion polymer and any optional excipients. In some embodiments, the spray solution comprises from 0.5-20 wt% of the active agent with a total solids (active agent, dispersion polymer, and any optional excipients) content of 2-40 wt%. In certain embodiments, the spray solution comprises 2.5-15 wt% active agent with a total solids content of 10-20 wt%. In some embodiments, the spray solution comprises at least 5 mg/mL active agent, such as from 5-167 mg/mL or from 5-125 mg/mL active agent.

The spray solution is sprayed to form a sprayed solid dispersion comprising the active agent, dispersion polymer, and ammonia. If an excipient is present in the spray solution, the SD further comprises the excipient. The SD also may comprise some residual solvent. Because the presence of ammonia in the sprayed solid dispersion may be undesirable, the ammonia is subsequently removed to form a product comprising a dispersion of the active agent and dispersion polymer. Residual solvent, if present, also may be removed. In some embodiments, the residual solvent and ammonia are concomitantly removed in a single process.

In some embodiments, the SD is formed by spray-drying and is a spray-dried dispersion (SDD). The term spray drying is used conventionally, and broadly refers to processes involving breaking up liquid mixtures into small droplets (atomization) and rapidly removing solvent from the mixture in a container (drying chamber) where there is a strong driving force for evaporation of solvent from the droplets. In general, active agent, dispersion polymer, and ammonia are added to a solvent to form a spray solution. The spray solution is then sprayed through an atomizer into a spray-drying chamber. The droplets are contacted in the spray-drying chamber with a heated drying gas such as dry nitrogen. Droplets dry rapidly, forming particles comprising a solid dispersion, or SDD, comprising the active and the dispersion polymer. The particles exit the spray dryer and are collected. Subsequent processes are used to remove ammonia and residual solvent from the particles.

Exemplary apparatus and procedures for forming SDDs are also described in U.S. Patent No. 8,263,128, U.S. Patent No.9,084,944, U.S. Patent No.9,248,584, U.S. Patent No. 9,724,664, and PCT Publication No. WO 2010/132827, each of which is incorporated herein by reference.

In some embodiments, the sprayed solid dispersion is a spray-layered dispersion (SLD) sprayed onto a core to provide a coating comprising a dispersion of the active agent and the dispersion polymer. For example, the spray solution may be sprayed through an atomizer onto cores to form a coating comprising a dispersion of the active agent and dispersion polymer on the cores. Exemplary cores include, but are not limited to, melt-congeal cores (e.g., as described in U.S. Publication No. 2010/0068276), nonpareil or sugar-based cores, or tablets.

### III. Solvent and Ammonia Removal

A sprayed solid dispersion made by a process as disclosed herein comprises an active agent, a dispersion polymer, and ammonia. The SD may further comprise one or more excipients. In many products comprising SDs, the presence of significant amounts of ammonia (e.g., in excess of 500 ppm) is undesirable. Thus, in some embodiments, the process further includes removal of residual ammonia from the SD to form a product comprising ≤ 500 ppm ammonia, such as a product comprising ≤ 400 ppm, ≤ 300 ppm, ≤ 200 ppm, ≤ 100 ppm, or even ≤ 50 ppm ammonia. In certain embodiments, the product comprises from 0-500 ppm, such as 1-500 ppm, 1-400 ppm, 1-300 ppm, 1-200 ppm, 1-100 ppm, 1-50 ppm, or 1-25 ppm ammonia. As ammonia is removed, the ammonia salt of the active agent is converted back to its free acid form in the SD. In some embodiments, at least 90 wt%, at least 95 wt%, at least 97 wt%, at least 98 wt%, or at least 99 wt% of the active agent is converted back to the free acid form as ammonia is removed from the sprayed solid dispersion. In some embodiments, from 90-100 wt%, 95-100 wt%, 97-100 wt%, 98-100 wt%, or 99-100 wt% of the active agent is converted back to the free acid form as ammonia is removed. If the dispersion polymer included acid groups, those groups also may be converted back to the acid form.

There is a correlation between the amount of ammonia remaining in the product and the molecular weight of the active agent. For instance, if sufficient ammonia is removed to convert 95 wt% of the active agent to its free acid form, a SD including a given mass of a low molecular weight active agent will retain a higher ppm level of ammonia than a SD including the same mass of a high molecular weight active agent. If the SD includes 100 g of an active agent having a molecular weight of 250 g/mol and 5 wt% remains as the ammonium salt, the SD will retain 0.02 mol ammonia. If the SD includes 100 g of an active agent having a molecular weight of 500 g/mol and 5 wt% remains as the ammonium salt, the SD will retain 0.01 mol ammonia.

In certain embodiments, the SD further comprises residual solvent. Inclusion of residual solvent may be undesirable. Separate processes may be used for removal of residual solvent and ammonia. Alternatively, at least a portion of the residual solvent and ammonia may be removed in a single process. In some embodiments, solvent removal occurs much more quickly than ammonia removal at the same conditions. In certain embodiments, all or substantially all (e.g., at least 95%) of the residual solvent is removed with the ammonia. For instance, methanol content may be reduced to less than 0.3 wt% in less than 30 minutes at temperature and relative humidity conditions that require 4-48+ hours for reduction of ammonia to levels ≤ 500 ppm. Thus, a single process may be performed with conditions selected to achieve a desired level of ammonia within a reasonable period of time.

In some embodiments, residual ammonia is removed by heating the SD at a temperature within a range of 30-70 °C for a sufficient period of time to form the product comprising ≤ 500 ppm ammonia. Advantageously, the temperature is selected such that appreciable thermal degradation of the active agent does not occur during the ammonia removal process. Residual solvent may be removed as the ammonia is removed. In some embodiments, the temperature is within a range of 40-60 °C. In some embodiments, the SD is maintained at 10-75% relative humidity (RH) during the ammonia and solvent removal process. In certain embodiments, the SD is maintained at 10-60% RH, 10-50% RH, 15-50% RH, or 15-30% RH.

Ammonia removal is performed for a period of time sufficient to reduce the ammonia content to ≤ 500 ppm. The ammonia content may be measured by any suitable method, such as with an ammonia ion selective electrode (ISE). In some embodiments, ammonia is measured by dissolving a known amount of the SD in dilute aqueous base (e.g., 0.1 M NaOH) and obtaining a millivolt reading with the ISE. The reading is compared to a standard curve prepared from solutions with known ammonia concentrations to determine the amount of ammonia remaining in the SD. In some embodiments, the period of time is from 2-60 hours, such as from 4-60 hours or 4-48 hours.

In some embodiments, removing residual ammonia further comprises blowing a sweep gas across the SD for at least a portion of the period of time. Desirably, the sweep gas is inert with respect to the SD. Suitable sweep gases include, but are not limited to, nitrogen, argon, carbon dioxide, air, and combinations thereof.

In some embodiments, the sweep gas further comprises water vapor (e.g., sufficient water to provide 10-50% RH during the ammonia and solvent removal process) and/or a volatile organic solvent vapor (e.g., methanol vapor). Without wishing to be bound by any theory or mechanism of action, inclusion of water vapor or volatile organic solvent vapor in the sweep gas may reduce the glass transition temperature of particles of the SD, thereby increasing the diffusion rate of residual ammonia and/or residual solvent in the particles. As a result, mass transfer of ammonia and/or residual solvent out of the particles may be increased, resulting in faster removal of the ammonia and/or residual solvent.

In some embodiments, the drying parameters (time, temperature, relative humidity, and/or sweep gas composition) may depend at least in part on the active agent pKa, the dispersion polymer composition, the identity of any excipients, mass ratios of the components, and/or particle morphology (e.g., particle size). For example, the time may increase as the particle size increases. Additionally, in at least some SDs, the dispersion polymer affects the time. For instance, it was found that a longer time was needed to remove ammonia from a SD comprising PVP-VA than from a comparable SD comprising HPMCAS. Increasing the temperature and/or RH may decrease the time to remove a desired amount of ammonia.

Ammonia (and solvent) removal may be performed using any suitable apparatus, e.g., a tray dryer, a mechanically agitated drying chamber, a drum dryer, a fluidized bed, a drying apparatus with a conveyor belt passing through the apparatus, and the like. The foregoing list is not intended to indicate that all embodiments are equivalent and/or equally suitable. In some embodiments, a tray dryer or a mechanically agitated drying chamber is utilized. When a mechanically agitated drying chamber is utilized, particles of the SD are introduced into a drying chamber having an external wall. The sweep gas is flowed through the drying chamber and the particles are circulated within the drying chamber by means of a mechanical agitator independent of the wall.

### IV. Active Agents

Embodiments of the disclosed process are used to form sprayed solid dispersions (SDs) comprising an active agent. By "active agent" is meant a drug, medicament, pharmaceutical, therapeutic agent, nutraceutical, agrochemical, fertilizer, pesticide, herbicide, nutrient, or other compound that may be desired to be formulated as a SD. The active agent may be a "small molecule," generally having a molecular weight of 2000 Daltons or less. In some embodiments, the SDs made by certain of the disclosed processes comprise two or more active agents.

Embodiments of the disclosed processes are particularly suitable for acidic active agents having a crystalline or free acid form pKa ≤ 7. In some embodiments, the free acid form of the active agent has a pKa within a range of 3-7.

The active agent may be a crystalline agent with a free acid form solubility in one or more C₁-C₃ alkanols of ≤ 40 mg/mL at ambient temperature (e.g., 20-25 °C). The term "crystalline" refers to solid material in which atoms or molecules are arranged in a definite pattern that is repeated regularly in three dimensions. In some embodiments, the active agent has a free acid form solubility in one or more C₁-C₅ alkanols of ≤ 30 mg/mL, ≤ 20 mg/mL, ≤ 10 mg/mL, ≤ 5 mg/mL, ≤ 2.5 mg/mL, or ≤ 1 mg/mL at ambient temperature. In certain embodiments, the active agent has a free acid form solubility in one or more C₁-C₃ alkanols within a range of 0.0001-40 mg/mL, 0.001-40 mg/mL, 0.001-30 mg/mL, 0.001-20 mg/mL, 0.001-10 mg/mL, 0.01-10 mg/mL, 0.1-10 mg/mL, 0.01-5 mg/mL, 0.01-2.5 mg/mL, or even 0.01-1 mg/mL at ambient temperature (e.g., a temperature of 20-25 °C).

### V. Dispersion Polymers

Embodiments of the disclosed process are used to form SDs comprising a dispersion polymer. In some embodiments, the SD comprises a plurality of dispersion polymers. Dispersion polymers suitable for use in the SDs formed by the disclosed methods should be inert, in the sense that they do not chemically react with the active agent in an adverse manner.

Suitable dispersion polymers include, but are not limited to, hydroxypropyl methylcellulose acetate succinate (HPMCAS), hydroxypropyl methylcellulose phthalate (HPMCP), hydroxypropyl methyl cellulose (HPMC), hydroxypropyl cellulose (HPC), cellulose acetate phthalate (CAP), carboxymethyl ethyl cellulose (CMEC), polyvinylpyrrolidone (PVP), poly(vinylpyrrolidone-co-vinyl acetate) (PVP-VA), poly(methacrylic acid-co-methyl methacrylate) (PMMAMA), poly(methacrylic acid-co-ethyl acrylate), or any combination thereof. Suitable PMMAMA polymers include, but are not limited to, poly(methacrylic acid-co-methyl methacrylate) 1:1 (Eudragit^{®} L100 polymer, Evonik Industries AG), and poly(methacrylic acid-co-methyl methacrylate) 1:2 (Eudragit^{®} S100 polymer). In some embodiments, the poly(methacrylic acid-co-ethyl acrylate) is poly(methacrylic acid-co-ethyl acrylate) 1:1. The foregoing list is not intended to indicate that all embodiments are equivalent and/or equally suitable. In some embodiments, the dispersion polymer comprises HPMCAS or PVP-VA.

### VI. Excipients

In some embodiments, the SDs further comprise one or more excipients in addition to the active agent and dispersion polymer(s). Suitable excipients include, but are not limited to, binders, fillers, diluents, disintegrants, glidants, surfactants, coatings, coloring agents, flavorants, and combinations thereof.

Exemplary surfactants include fatty acids, alkyl sulfonates, and commercial surfactants such as those sold under tradenames such as benzethanium chloride (Hyamine^{®} 1622, available from Lonza, Inc., Fairlawn, NJ, docusate sodium (available from Mallinckrodt Spec. Chem., St. Louis, MO, and polyoxyethylene sorbitan fatty acid esters (Tween^{®} surfactant, available from ICI Americas Inc., Wilmington, DE, Liposorb^{®} P-20, available from Lipochem Inc., Patterson, NJ, and Capmul^{®} POE-0, available from Abitec Corp., Janesville, WI), sorbitan esters (such as Span^{®} 20 (sorbitan monolaurate), 40 (sorbitan monopalmitate), 60 (sorbitan monostearate), 65 (sorbitan tristearate), 80 (sorbitan monooleate) and 85 (sorbitan trioleate) nonionic surfactants, available from Sigma-Aldrich, St. Louis, MO), and natural surfactants such as sodium taurocholic acid, 1-palmitoyl-2-oleoyl-sn-glycero-3-phosphocholine, lecithin, and other phospholipids and mono- and diglycerides. Examples of binders include methyl cellulose, microcrystalline cellulose, starch, and gums such as guar gum, and tragacanth. Examples of glidants include talc, colloidal silica, magnesium stearate and calcium stearate. Examples of disintegrants include sodium starch glycolate, sodium alginate, carboxymethyl cellulose sodium, methyl cellulose, and croscarmellose sodium. Examples of other matrix materials, fillers, or diluents include lactose, mannitol, xylitol, microcrystalline cellulose, calcium diphosphate, calcium silicate, hydrated aluminum silicate, bentonite, hectorite, montmorillonite, magnesium trisilicate, magnesium oxide, and starch.

### VII. Sprayed solid dispersions

Sprayed solid dispersions made by embodiments of the disclosed method comprise an active agent, a dispersion polymer, and ≤ 500 ppm ammonia. In some embodiments, the SD comprises ≤ 400 ppm, ≤ 300 ppm, ≤ 200 ppm, ≤ 100 ppm, or even ≤ 50 ppm ammonia.

In some embodiments, the active agent is an acidic agent with a pKa ≤ 7 and a free acid form solubility ≤ 10 mg/mL in methanol, ethanol, n-propanol, isopropanol, or any combination thereof. In some embodiments, the SD comprises at least 0.5 wt% of the active agent, such as at least 1 wt%, at least 5 wt%, at least 10 wt%, at least 25 wt%, or at least 50 wt% of the active agent. In certain embodiments, the SD comprises 0.5-95 wt% of the active agent, such as 1-95 wt%, 5-95 wt%, 10-95 wt%, or 25-95 wt% of the active agent.

In some embodiments, at least 90 wt% of the active agent in the SD is in its free acid form. In certain embodiments, at least 95 wt%, at least 97 wt%, at least 98 wt%, or at least 99 wt% of the active agent is in its free acid form. For example, from 90-100 wt%, 95-100 wt%, 97-100 wt%, 98-100 wt%, or 99-100 wt% of the active agent may be in its free acid form in the sprayed dispersion.

The SD further comprises a dispersion polymer. In some embodiments, the dispersion polymer comprises hydroxypropyl methylcellulose acetate succinate (HPMCAS), hydroxypropyl methylcellulose phthalate (HPMCP), hydroxypropyl methyl cellulose (HPMC), hydroxypropyl cellulose (HPC), cellulose acetate phthalate (CAP), carboxymethyl ethyl cellulose (CMEC), polyvinylpyrrolidone (PVP), poly(vinylpyrrolidone-co-vinyl acetate) (PVP-VA), poly(methacrylic acid-co-methyl methacrylate) 1:1, poly(methacrylic acid-co-methyl methacrylate) 1:2, poly(methacrylic acid-co-ethyl acrylate) 1:1, or any combination thereof. In certain embodiments, the SD comprises two or more dispersion polymers. The SD may further comprise one or more excipients.

In some embodiments, the SD comprises, consists essentially of, or consists of the active agent, the dispersion polymer(s), ≤ 500 ppm ammonia and no more than trace amounts (≤ 50 ppm) solvent. "Consists essentially of" means that the SD does not include components that materially affect the properties of the SD. Thus, the SD does not include, for example, polymers other than the dispersion polymer, or an active agent with a pKa > 7. However, the SD may include trace amounts (e.g., < 500 ppm) of other components that do not materially affect the SD properties, such as minor amounts of impurities present in the materials used to make the SD. In certain embodiments, the SD comprises, consists essentially of, or consists of the active agent, the dispersion polymer(s), ≤ 50 ppm ammonia and ≤ 5 ppm solvent. In an independent embodiment, the SD comprises, consists essentially of, or consists of the active agent, the dispersion polymer(s), one or more excipients, ≤ 500 ppm ammonia and no more than trace amounts (≤ 50 ppm) solvent. In another independent embodiment, the SD comprises, consists essentially of, or consists of the active agent, the dispersion polymer(s), one or more excipients, ≤ 50 ppm ammonia and ≤ 5 ppm solvent.

The active agent in the SD may be crystalline, amorphous, or any state in-between. In certain embodiments, the active agent in the SD is amorphous or substantially (i.e., at least 90 wt%) amorphous. In one embodiment, the active agent is molecularly dispersed throughout the SD. In an independent embodiment, the SD is a spray-dried dispersion comprising a plurality of particles, wherein at least some of the particles include domains of active agent. The active agent domains may comprise amorphous active agent, crystalline active agent, or a combination of amorphous and crystalline active agent. In some embodiments, at least 90 wt%, such as 90-100 wt%, 90-99.9 wt%, or 90-99.5 wt%, of the active agent in the active agent domains is amorphous.

In some embodiments, a SD prepared by the disclosed method has advantages compared to SDs prepared by other methods, such as SDs comprising an active agent salt prepared with a nonvolatile counterion, e.g., Na⁺ or K⁺, or SDs prepared by spraying the free acid form of the active agent. For instance, embodiments of the SDs disclosed herein may have a higher potency and/or a greater physical stability (e.g., decreased hygroscopicity) relative to a SD comprising a salt of the active agent. In some embodiments, embodiments of the SDs include a greater percentage of the active agent compared to SDs prepared from the free acid form of the active agent where solubility constraints limit the concentration of the active agent in the spray solution and subsequent solid dispersion.

### VII. Representative Embodiments

Several representative embodiments are set forth in the following paragraphs.

A method for preparing a sprayed solid dispersion, comprising: combining an active agent, a dispersion polymer, an amount of ammonia, and a solvent to form a spray solution, wherein the solvent comprises a C₁-C₃ alkanol, the active agent, in a free acid form, has a pKa ≤ 7 and a solubility ≤ 40 mg/mL in the solvent, and the amount of ammonia is sufficient to solubilize the active agent in the solvent; spraying the spray solution to form a sprayed solid dispersion comprising the active agent, the dispersion polymer, and ammonia; and removing residual ammonia from the sprayed solid dispersion to form a product comprising a solid dispersion of the active agent and the dispersion polymer, the product comprising ≤ 500 ppm ammonia.

The method of the foregoing paragraph, wherein the amount of ammonia is at least 0.95 molar equivalents relative to a sum of acid groups on the active agent equivalents relative to a sum of acid groups on the active agent.

The method of the first paragraph, wherein the amount of ammonia is at least 0.95 molar equivalents relative to a sum of acid groups on the active agent and any acid groups on the dispersion polymer.

The method of the foregoing paragraph, wherein the amount of ammonia is from 0.95 to 4 molar equivalents relative to a sum of acid groups on the active agent and any acid groups on the dispersion polymer.

The method of the first paragraph, wherein the amount of ammonia is at least 0.95 molar equivalents relative to a sum of acid groups on an amount of the active agent that exceeds the free acid form solubility of the active agent in the solvent and any acid groups on the dispersion polymer.

The method of any of the foregoing paragraphs, wherein at least 90 wt% of the active agent in the product is in the free acid form.

The method of any of the foregoing paragraphs, wherein the C₁-C₃ alkanol comprises methanol, ethanol, n-propanol, isopropanol, or any combination thereof.

The method of any of the foregoing paragraphs, wherein the solvent comprises the C₁-C₃ alkanol and a co-solvent.

The method of the foregoing paragraph, wherein the co-solvent comprises water, tetrahydrofuran, ethyl acetate, methyl acetate, 1 ,3-dioxolane, acetone, methyl ethyl ketone, or any combination thereof.

The method of either of the foregoing paragraphs, wherein the solvent comprises 0.01-30 wt% water, with the remainder being the C₁-C₃ alkanol.

The method of any of the foregoing paragraphs, wherein the active agent has a solubility in the spray solution at least 2-fold higher than a solubility of a free acid form of the active agent in the solvent without ammonia.

The method of any of the foregoing paragraphs, wherein the spray solution has a dissolved solids content within a range of 2-40 wt%, wherein 0.5-95 wt% of the dissolved solids is the active agent.

The method of any of the foregoing paragraphs, wherein the spray solution comprises 0.5-20 wt% of the active agent.

The method of any of the foregoing paragraphs, wherein removing residual ammonia comprises heating the sprayed solid dispersion at a temperature within a range of 30-70 °C and a relative humidity (RH) within a range of 10-75% for a sufficient period of time to form the product comprising ≤ 500 ppm ammonia.

The method of the foregoing paragraph, wherein: (i) the period of time is from 2 to 60 hours; or (ii) the temperature is within a range of 40-60 °C; or (iii) the RH is within a range of 15-50%; or (iv) any combination of (i), (ii), and (iii).

The method of any of the foregoing paragraphs, wherein removing residual ammonia further comprises blowing a sweep gas across the sprayed solid dispersion while heating the sprayed solid dispersion at the temperature and RH.

The method of the foregoing paragraph, wherein the sweep gas comprises nitrogen, argon, carbon dioxide, air, or any combination thereof.

The method of the foregoing paragraph, wherein the sweep gas further comprises water vapor or a volatile organic solvent vapor.

The method of the foregoing paragraph, wherein the sweep gas further comprises a volatile organic solvent vapor, and the volatile organic solvent is methanol.

The method of any of the foregoing paragraphs, wherein removing residual ammonia is performed in a tray dryer or a mechanically agitated drying chamber.

The method of any of the foregoing paragraphs, wherein at least a portion of the residual solvent is removed simultaneously with removing residual ammonia from the sprayed solid dispersion.

The method of any of the foregoing paragraphs, wherein the dispersion polymer has a dry glass transition temperature T_{g} > 60 °C as measured by differential scanning calorimetry.

The method of any of the foregoing paragraphs, wherein the dispersion polymer comprises hydroxypropyl methylcellulose acetate succinate (HPMCAS), hydroxypropyl methylcellulose phthalate (HPMCP), hydroxypropyl methyl cellulose (HPMC), hydroxypropyl cellulose (HPC), cellulose acetate phthalate (CAP), carboxymethyl ethyl cellulose (CMEC), polyvinylpyrrolidone (PVP), poly(vinylpyrrolidone-co-vinyl acetate) (PVP-VA), poly(methacrylic acid-co-methyl methacrylate), poly(methacrylic acid-co-ethyl acrylate), or any combination thereof.

The method of any of the foregoing paragraphs, wherein the spray solution comprises a plurality of dispersion polymers.

The method of any of the foregoing paragraphs, wherein the spray solution further comprises one or more excipients.

The method of the foregoing paragraph, where the one or more excipients comprise one or more binders, fillers, diluents, disintegrants, glidants, surfactants, coatings, coloring agents, flavorants, or any combination thereof.

The method of either of the foregoing paragraphs, wherein the amount of ammonia is at least 0.95 molar equivalents relative to a sum of acid groups on the active agent, any acid groups on the dispersion polymer, and any acid groups on the one or more excipients.

The method of the foregoing paragraph, wherein the amount of ammonia is from 0.95 to 4 molar equivalents relative to the sum of acid groups on the active agent, any acid groups on the dispersion polymer, and any acid groups on the one or more excipients.

A sprayed solid dispersion prepared by the method of any of the foregoing paragraphs, wherein the sprayed solid dispersion comprises at least 0.5 wt% of the active agent; and ≤ 500 ppm ammonia.

The sprayed solid dispersion of the foregoing paragraph, wherein at least 90 wt% of the active agent in the sprayed solid dispersion is in a free acid form.

The sprayed solid dispersion of either of the foregoing paragraphs, comprising 0.5-95 wt% of the active agent, wherein at least 90 wt% of the active agent is amorphous; the dispersion polymer; and ≤ 50 ppm ammonia.

The method of any of the foregoing paragraphs, wherein the product comprises ≤ 300 ppm ammonia, ≤ 100 ppm ammonia, or ≤ 50 ppm ammonia.

### VIII. Examples

### Methods of characterization:

**GC Analysis:** Headspace gas chromatography (GC) was used to quantify the amounts of residual methanol present in test samples. GC samples were removed from the tray dryer at regular time intervals, and a 10-60mg sample of known mass sealed hermetically in a 20 mL headspace vial. Samples were dissolved in 4 mL of N-N-dimethylacetamide injected through the PTFE/silicone septa. An Agilent^{®} G7890 GC (Agilent Technologies, Santa Clara, CA) equipped with automated headspace sampler was used with a DB-624 column (30 m x 0.32mm ID x 1.8µm) and a flame ionization detector. The residual solvent present in a sample is quantitated using a 6-point standard curve of headspace samples with known solvent content.

**PXRD:** Powder X-ray diffraction (PXRD) was performed on a Rigaku^{®} Miniflex^{®} 600 X-ray diffractometer (Rigaku Corporation, Tokyo, Japan) equipped with a D/tex high speed linear detector operated in theta/2-theta mode. Samples were spread onto a 0.5 mm zero background holder. Analyses were performed using Cu K-alpha radiation at 600 W (40kV and 15mA), and with a 1.25° dispersive slit, 8.0 mm scattering slit, and 5° soller slits on both the incident and diffracted beams, and a 15 micron Ni foil to filter the Cu K-beta diffracted beam. The samples were scanned from 3° to 40° 2θ with 0.02° 2θ step size at 2.5°/min.

**Ammonia Quantitation** - **Ion Selective Electrode (ISE) Method:** Ammonia quantitation in the SDD samples was accomplished using an ammonia ion selective electrode, Orion^{®} 9512HPBNWP (ThermoFisher Scientific), and a Beckman Coulter model PHI410 meter. The ISE was calibrated using ammonium sulfate primary standard from spectrum chemical. Aqueous solutions of known ammonia concentration in 0.1 M sodium hydroxide were measured as millivolts and the values were used to generate a linear standard curve. SDD samples of known mass were dissolved in 0.1 molar sodium hydroxide and then analyzed using the ISE. The millivolt readings were recorded and then converted to concentration. Ammonia concentrations in the SDD are reported as part per million (ppm).

### Example 1

### Sulfasalazine Concentration Enhancement in Methanol with Ammonia

A saturated solution of sulfasalazine (Spectrum Chemical) was prepared in methanol with excess crystalline sulfasalazine. After centrifugation, analysis of the supernatant by thermogravimetric methods gave a crystalline solubility of about 1.0 mg/mL in methanol.

A second mixture of sulfasalazine in methanol was prepared by stirring 656.0 mg of sulfasalazine (1.65 mmol) in 14.00 mL of methanol. To this slurry, 0.825 mL of 2.14 M ammonia (1.76 mmol) in methanol was added with stirring resulting in complete dissolution of sulfasalazine within 1 minute. The sulfasalazine concentration is greater than 43 mg/mL in methanol. The observed solubility enhancement of the sulfasalazine in methanol in the presence of ammonia was about 43-fold.

### Example 2

### Piroxicam Concentration Enhancement in Methanol with Ammonia

A saturated solution of piroxicam (Spectrum Chemical) was prepared in methanol with excess crystalline piroxicam. After centrifugation, analysis of the supernatant by thermogravimetric methods gave a crystalline solubility of about 2.1 mg/mL in methanol.

A second mixture of piroxicam in methanol was prepared by stirring 661.8 mg of piroxicam (2.00 mmol) in 15.00 mL of methanol. To this slurry, 1.0 mL of 2.14 M ammonia (2.14 mmol) in methanol (was added with stirring resulting in a clear yellow solution within 1 minute. The piroxicam concentration is greater than 41 mg/mL in methanol. The observed solubility enhancement of the piroxicam in methanol in the presence of ammonia was greater than 19-fold.

### Example 3

### Piroxicam/PVP-VA65 SDD with Ammonia as Processing Aid

A spray solution was prepared by dissolving 16.02 grams of Kollidon^{®} VA-64 vinylpyrrolidone-vinyl acetate copolymer (BASF Corp.) into 475.07 g of methanol. To this solution 4.00g, 12.07 mmol, of piroxicam was added, resulting in a suspension of the piroxicam. To the suspension, 6.00 mL of 2.14 M (12.84 mmol) ammonia in methanol (Aldrich Chemical) was added with stirring, resulting in a clear yellow solution.

The solution was pumped into a lab-scale spray drying chamber using head pressure of 150 psi on a solution pot. The solution flow rate was estimated to be 30 g/min. The drug/polymer solution was atomized through a Schlick model 121 size 2.0 pressure-swirl nozzle. Heated nitrogen gas was introduced into the 0.3-m diameter stainless steel chamber at a temperature of 139°C and flow rate of 500 g/min. The outlet temperature of the gas exiting the chamber was 45°C. The solid dispersion was collected using a cyclone to separate the solid particles from the gas stream, and was stored in a sealed container at 2-8°C.

### Example 4

### Piroxicam/HPMCAS SDD with Ammonia as Processing Aid

A spray solution was prepared by dissolving 16.00 g of HPMCAS-M (Shin-Etsu AQOAT^{®}) into 468.01 g of methanol. To this solution 4.00 g, 12.07 mmol, of piroxicam was added resulting in a suspension of the active agent. To the suspension, 14.82 mL of 2.14 M (31.71 mmol) ammonia in methanol (Aldrich Chemical) was added with stirring resulting in a slightly hazy yellow solution. This amount of ammonia was used to neutralize both the active agent and the polymer.

The solution was pumped into a lab-scale spray drying chamber using head pressure of 150 psi on a solution pot. The solution flow rate was estimated to be 30 g/min. The drug/polymer solution was atomized through a Schlick model 121 size 2.0 pressure-swirl nozzle. Heated nitrogen gas was introduced into the 0.3-m diameter stainless steel chamber at a temperature of 139°C and flow rate of 500 g/min. The outlet temperature of the gas exiting the chamber was 45°C. The solid dispersion was collected using a cyclone to separate the solid particles from the gas stream, and was stored in a sealed container at 2-8°C.

### Examples 5-10

### Ammonia Removal

Ammonia was removed from the spray dried dispersions using an ES2000 environmental chamber from Environmental Specialties, Inc. at three different conditions. Constant temperature and relative humidity (RH) of the drying chamber were maintained for the three conditions tested: 40°C and 15%RH, 50°C and 30%RH, and 60°C and 30% RH.

At each set of conditions, samples of the spray-dried dispersions were removed from the environmental chamber at time intervals of 0 hours, 4 hours, 8 hours, 24 hours, and 48 hours. The samples were stored in sealed vials for residual ammonia analysis using an ion selective electrode as described above. The results are shown in Table 1.

**Table 1**

| Ex. | SDD | NH₃ Removal Conditions | ppm NH₃ 0 hours | ppm NH₃ 4 hours | ppm NH₃ 8 hours | ppm NH₃ 24 hours | ppm NH₃ 48 hours |
|---|---|---|---|---|---|---|---|
| 5 | Piroxicam | 40 °C | 6550 | 3630 | 3010 | 1560 | 1080 |
| | PVP-VA 64 | 15%RH | | | | | |
| 6 | Piroxicam | 40 °C | 4850 | 760 | 390 | 70 | 34 |
| | HPMCAS | 15%RH | | | | | |
| 7 | Piroxicam | 50 °C | 5640 | 2480 | 1910 | 546 | 185 |
| | PVP-VA 64 | 30%RH | | | | | |
| 8 | Piroxicam | 50 °C | 4540 | 320 | 100 | 14 | 10 |
| | HPMCAS | 30%RH | | | | | |
| 9 | Piroxicam | 60 °C | 5720 | 580 | 160 | 47 | 33 |
| | PVP-VA 64 | 30%RH | | | | | |
| 10 | Piroxicam | 60 °C | 4750 | 24 | 10 | 10 | 10 |
| | HPMCAS | 30%RH | | | | | |

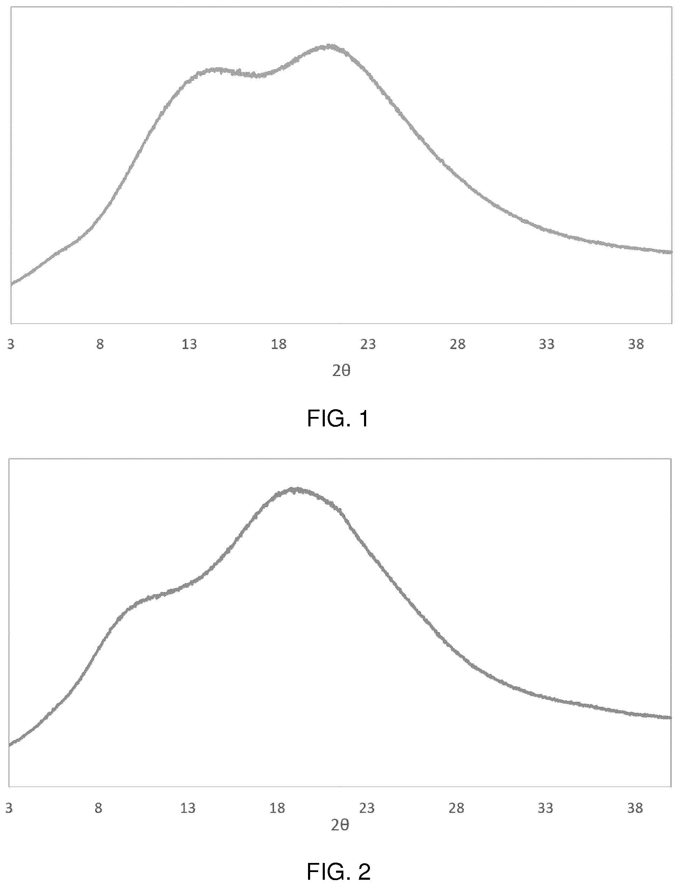

FIG. 1 is a PXRD spectrum of the SDD of Example 7 after 48 hours at 50 °C, 30% RH. FIG. 2 is a PXRD spectrum of the SDD of Example 8 after 48 hours at 50 °C, 30% RH. The lack of sharp peaks in the spectra suggests that the samples are amorphous.

### Example 11

### Solvent Removal

To demonstrate the significant differences in the time and conditions required for removing solvent and ammonia for the SDs, a second set of piroxicam SDDs containing either HPMCAS or PVPVA-64 polymer were manufactured using the same procedures and quantities in Examples 3 and 4. The wet SDDs were immediately transferred into the ES2000 environmental chamber set to 40°C/15%RH. GC samples were taken at time points of 0, 0.5, 1, 2, and 4 hours. The initial solvent content of the SDDs was about 1-2 wt% methanol. No methanol was detected in the samples for all time points 0.5 hr and greater, indicating the methanol has been removed well below ICH (International Council for Harmonisation of Technical Requirements for Pharmaceuticals for Human Use) specification of 0.3 wt% within 30 minutes at these drying conditions.

Although the same equipment was used to remove residual solvent and residual ammonia from the SDD, it is clear that there are significant differences in the conditions and processing times required to achieve acceptable low levels of each. For example, the removal of the methanol from SDDs prepared by the method in Examples 3 and 4 was monitored, and in both cases the residual methanol was well below the ICH specification of 0.3 wt% after just 30 minutes in the dryer at 40 °C/15% RH. At these conditions, removal of ammonia to a target value of less than 500 ppm ammonia is more than 8 times slower than the time required to remove the methanol from the HPMCAS SDD (Table 1, Example 6). In the case of the PVP-VA 64 SDD, both longer time, > 24hr and higher temperature/humidity (50 °C/30% RH) conditions are required (Table 1, Example 7). This amounts to more than 48 times longer than the methanol removal at 40 °C /15% RH. To reach a more preferred target value of less than 100 ppm ammonia in the SDD, the time required with the HPMCAS SDD approaches 24 hours at 40 °C/15% RH (Table 1, Example 6) which is 48 times longer than methanol removal. For the PVP-VA SDD, reaching a value below 100 ppm ammonia using 50 °C/30% RH conditions (Table 1, Example 7) would require well beyond 48 hours of processing which is 98 times longer than the methanol removal. To bring processing times for PVP-VA SDDs within a 24 hour period, processing conditions must be increased to 60 °C/30% RH (Table 1, Example 9), which is far higher than what is required for methanol removal.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

## Claims

1. A method for preparing a sprayed solid dispersion, comprising:
combining an active agent, a dispersion polymer, an amount of ammonia, and a solvent to form a spray solution, wherein
the solvent comprises a C₁-C₃ alkanol, wherein the C₁-C₃ alkanol is methanol, ethanol, n-propanol, isopropanol, or any combination thereof,
the active agent, in a free acid form, has a pKa ≤ 7 and a solubility ≤ 40 mg/mL in the solvent at a temperature of 20 °C to 25 °C, and
the amount of ammonia is sufficient to solubilize the active agent in the solvent and is present in an amount of:
- at least 0.95 molar equivalents relative to a sum of acid groups on the active agent; or
- at least 0.95 molar equivalents relative to a sum of acid groups on the active agent and any acid groups on the dispersion polymer; or
- from 0.95 to 10 molar equivalents relative to a sum of acid groups on the active agent and any acid groups on the dispersion polymer; or
- at least 0.95 molar equivalents relative to a sum of acid groups on an amount of the active agent that exceeds the free acid form solubility of the active agent in the solvent and any acid groups on the dispersion polymer;
spraying the spray solution to form a sprayed solid dispersion comprising the active agent, the dispersion polymer, and ammonia; and
removing residual ammonia from the sprayed solid dispersion by heating the sprayed solid dispersion for a period of time sufficient to form a product comprising a solid dispersion of the active agent and the dispersion polymer, the product comprising ≤ 500 ppm ammonia.

2. The method of claim 1, wherein removing residual ammonia from the sprayed solid dispersion by heating the sprayed solid dispersion is performed at a temperature such that appreciable thermal degradation of the active agent does not occur.

3. The method of claim 1 or 2, wherein at least 90 wt% of the active agent in the product is in the free acid form.

4. The method of any one of claims 1-3, wherein the solvent further comprises a co-solvent.

5. The method of claim 4, wherein the co-solvent comprises water, tetrahydrofuran, ethyl acetate, methyl acetate, 1,3-dioxolane, acetone, methyl ethyl ketone, or any combination thereof.

6. The method of any one of claims 1-5, wherein the active agent has a solubility in the spray solution at least 2-fold higher than a solubility of a free acid form of the active agent in the solvent without ammonia.

7. The method of any one of claims 1-6, wherein the spray solution has a dissolved solids content within a range of 2-40 wt%, wherein 0.5-95 wt% of the dissolved solids is the active agent.

8. The method of any one of claims 1-7, wherein the spray solution comprises 0.5-20 wt% of the active agent.

9. The method of any one of claims 1-7, wherein the period of time is from 2 to 60 hours.

10. The method of claim 1 1, wherein removing residual ammonia further comprises blowing a sweep gas across the sprayed solid dispersion while heating the sprayed solid dispersion, wherein the sweep gas comprises nitrogen, argon, carbon dioxide, air, or any combination thereof.

11. The method of claim 10, wherein the sweep gas further comprises water vapor or a volatile organic solvent vapor.

12. The method of any one of claims 1-11, wherein at least a portion of the residual solvent is removed simultaneously with removing residual ammonia from the sprayed solid dispersion.

13. The method of any one of claims 1-12, wherein the dispersion polymer has a dry glass transition temperature Tg > 60 °C as measured by differential scanning calorimetry.

14. The method of any one of claims 1-13, wherein the dispersion polymer comprises hydroxypropyl methylcellulose acetate succinate (HPMCAS), hydroxypropyl methylcellulose phthalate (HPMCP), hydroxypropyl methyl cellulose (HPMC), hydroxypropyl cellulose (HPC), cellulose acetate phthalate (CAP), carboxymethyl ethyl cellulose (CMEC), polyvinylpyrrolidone (PVP), poly(vinylpyrrolidone-co-vinyl acetate) (PVP-VA), poly(methacrylic acid-co-methyl methacrylate), poly(methacrylic acid-co-ethyl acrylate), or any combination thereof.

15. The method of any one of claims 1-14, wherein:
the spray solution further comprises one or more excipients; and
the amount of ammonia is at least 0.95 molar equivalents relative to a sum of acid groups on the active agent, any acid groups on the dispersion polymer, and any acid groups on the one or more excipients.
